# FASCICULE DE BREVET EUROPEEN

(11) **EP 0 497 672 B1**
(45) Date de publication et mention de la délivrance du brevet: **06.11.1996**
(21) Numéro de dépôt: 92400191.0
(22) Date de dépôt: 24.01.1992
(51) Int. Cl.: A61N 1/40

(54) **Casque émetteur d'ondes électromagnétiques hautes fréquences pulsées à effets athermiques**
Helm zur Sendung von auf Hochfrequenz pulsierenden elektromagnetischen Wellen mit athermischen Effekten
Helmet generating high frequency pulsed electromagnetical waves with athermic effects

(30) Priorité: 30.01.1991 FR 9101293
(43) Date de publication de la demande: 05.08.1992
(73) Titulaire: Mazaury, André, 13250 Saint Chamas (FR); Nguyen, Christian, 03000 Montluçon (FR); Bellisi, Vincente, 13250 Saint Chamas (FR)
(72) Inventeur: Mazaury, André, 13250 Saint Chamas (FR); Nguyen, Christian, 03000 Montluçon (FR)

(56) Documents cités:
- FR-A- 1 350 890
- FR-A- 2 454 817

## Description

La présente invention concerne un dispositif émetteur d'ondes électromagnétiques hautes fréquences pulsées à effets athermiques, pour le traitement du crâne d'un individu pour applications médicales, esthétiques, ou de soins capillaires.

Traditionnellement, les problèmes capillaires ou sanguins du cuir chevelu sont traités par des crèmes ou huiles répandues sur la surface à traiter ou par absorption médicamenteuse pouvant provoquer des problèmes d'intolérances ou d'inflammations chez le patient.

L'application des ondes électromagnétiques hautes fréquences pulsées athermiques est connue pour provoquer une activation du processus de réparation tissulaire ou pour une modification positive dans le cas d'un désordre cellulaire, sans provoquer d'effets thermiques par le caractère pulsé des ondes.

FR-A-245817 décrit le préambule de la revendication 1: le document décrit un appareil de traitement à ondes électromagnétiques pulsées à effet athermique à intensité faible, utilisant une antenne destinée à être placée au contact des tissus à traiter dont les impulsions sont soumises à un balayage selon un rythme accordé sur un rythme biologique.

FR-A-1350890 décrit un casque pour sécher les cheveux permettant d'appliquer un champ électrique haute fréquence aux cheveux humides dans le but de faire évaporer l'eau par chauffage haute fréquence. Le champ électrique haute fréquence est créé à partir de deux électrodes, l'une étant fixée à l'intérieur du casque et l'autre, en forme de peigne étant glissée dans les cheveux, les cheveux étant donc interposés entre les deux électrodes.

La présente invention a pour objet un dispositif émetteur d'ondes électromagnétiques hautes fréquences pulsées à effets athermiques pour le traitement du crâne d'un individu, comme défini dans la revendication 1.

Le dispositif selon l'invention permet de traiter la surface de la boîte crânienne sans besoin d'adjuvant, et sans contact avec la surface à traiter, cela par l'émission d'ondes électromagnétiques à hautes fréquences pulsées athermiques, de faible puissance d'émission due à l'application directe et localisée sur la zone à traiter.

Selon l'invention, la puissance d'émission peut être uniformément répartie suivant la surface et le choix de l'homme de l'art sur le dessin du conducteur de l'antenne en fonction de la zone à traiter.

Le dispositif conforme à l'invention peut être aussi mis en émission sur la tête du patient avec les cheveux humides ou avec un produit quel qu'il soit, cela du au caractère d'ondes hautes féquences n'ayant besoin pour se propager sur la surface à traiter, d'être en contact et ne pouvant provoquer d'électrocution.

Afin d'aider à la compréhension des caractéristiques techniques de la présente invention, nous décrivons une réalisation, qui, celle-ci n'est présentée qu'à titre d'exemple non limitatif quant à la forme ou à la mise en oeuvre de ses applications.

Nous prendrons comme références les figures suivantes:
- **La figure 1**: une vue générale en coupe du dispositif conforme à l'invention
- **La figure 2**: une vue de face du dispositif conforme à l'invention

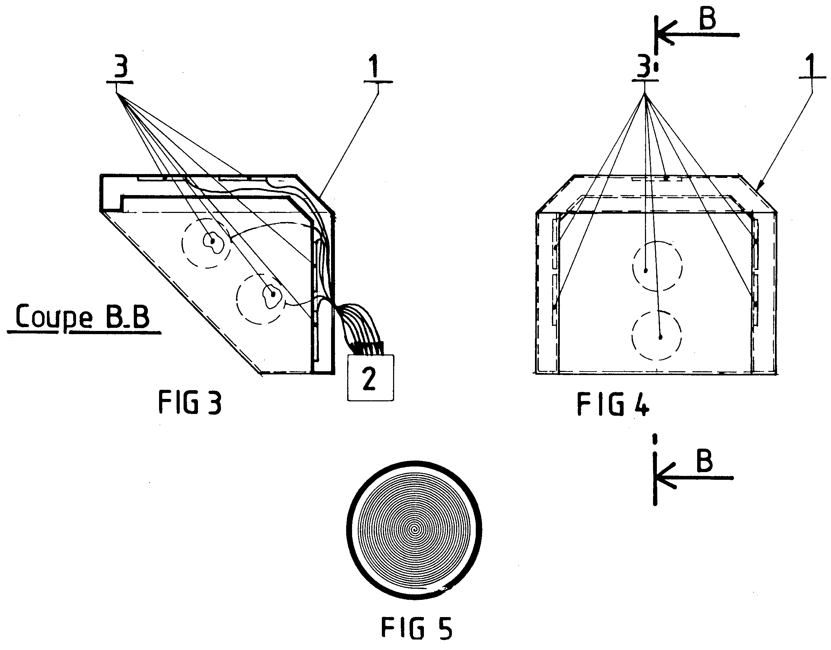

- **La figure 3**: une vue en coupe du casque conforme à l'invention
- **La figure 4**: une vue de face du casque conforme à l'invention
- **La figure 5**: une vue de l'antenne d'émission suivant l'invention
- **La figure 6**: une vue du circuit d'émission avec ses trois modules et leurs schémas respectifs conformément à l'invention
- **La figure 7**: une vue de l'oscillogramme du signal émis par le module A
- **La figure 8**: une vue de l'oscillogramme du signal émis par le module B
- **La figure 9**: une vue de l'oscillogramme du signal émis par le module C

Le dispositif comprend un casque de taille à envelopper la boîte crânienne, ce casque ayant pout but de supporter et de positionner au niveau de la boîte crânienne une ou plusieurs antennes **(3)** pour l'émission du champ électromagnétique de haute fréquence.

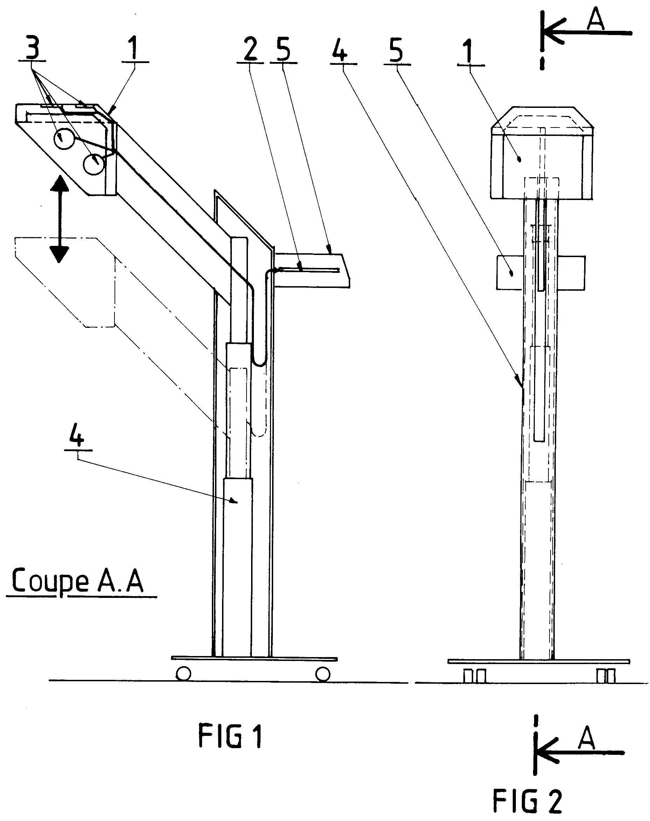

Comme on voit sur les figures **1** et **2**, le casque est relié à un bras pouvant se déplacer verticalement à l'aide d'un système mécanique **(4)** ou électrique commandé manuellement ou automatiquement.

Le casque peut se positionner au niveau de la boîte crânienne sans contact avec celle-ci.

Le dispositif comprend un boîtier de commande **(5)** qui comprend la partie de commande de sélection de fréquences et réglage de puissance d'émission.

Le dispositif comprend un circuit **(2)** produisant des signaux électriques impulsionnels à hautes fréquences pulsées, ce circuit est relié à une ou plusieurs antennes **(3)** réalisées dans un matériau conducteur pour l'émission du champ, l'ensemble est relié à une alimentation électrique extérieure ou, pour des raisons d'autonomie à une batterie ou autres principes d'alimentations autonomes.

Le casque, pour des raisons d'émission optimale, est constitué d'un matériau supportant le conducteur **(3)** produisant l'émission, le casque devenant ainsi l'antenne à part entière.

Le dispositif peut aussi comprendre plusieurs circuits électroniques **(2)** produisant des signaux électriques impulsionnels à haute fréquence pulsées relié chacun à leur propre antenne **(3)** pour l'émission du champ.

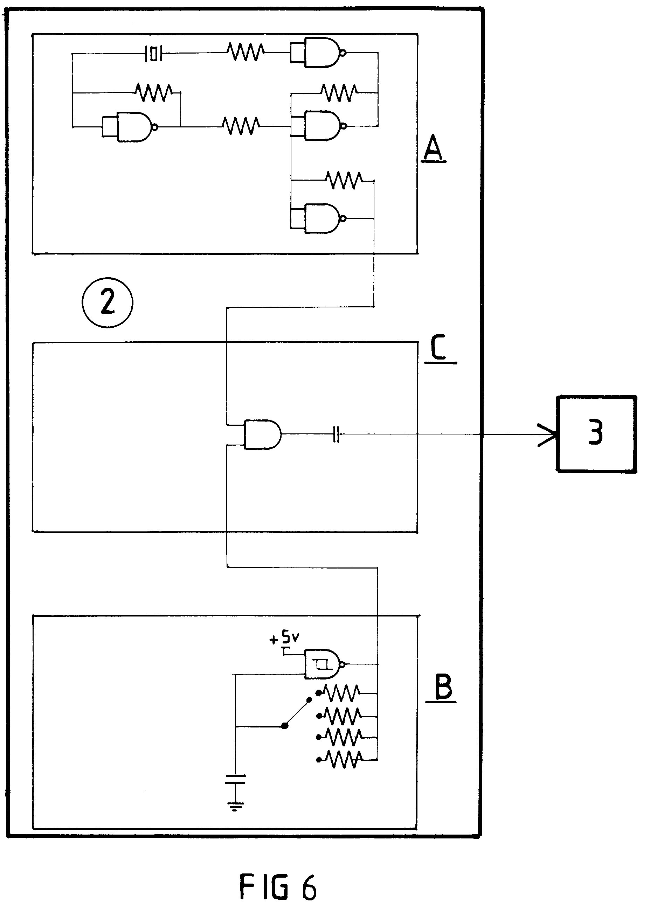

Le principe de fonctionnement du circuit **(2)** est basé sur l'association de trois modules distincts **(A) (B) (C) (figure 6)** réunis sur un même circuit électronique intégré au boîtier de commande **(5)**.

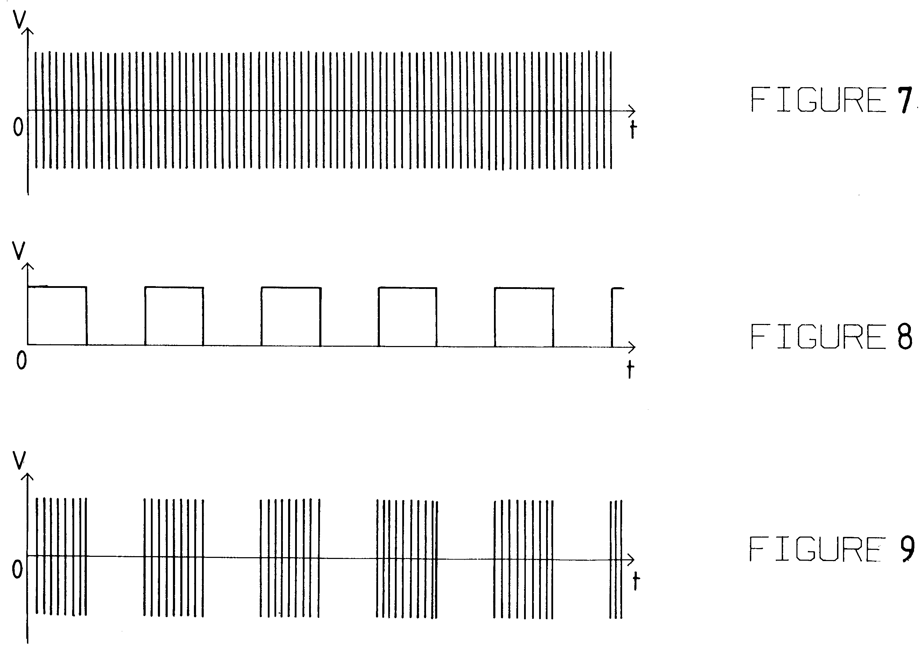

Le premier module **(A)** est un module d'émission haute fréquence de type sinusoïdal **(figure 7)** de fréquence 27.120 Mhz équivalent environ à une bande de 11,06 mètres, fréquence réservée à l'utilisation médicale, mais pour le dispositif suivant l'invention cette fréquence n'est pas restrictive, constituée d'un cristal de quartz entrant en résonnance par un dispositif électronique.

Le second module **(B)** est un module d'émission basse fréquence de type signal carré **(figure 8)** ayant un rapport cyclique approximativement de 50%, généré électroniquement. Cette fréquence allant de 5 hertz à 10000 hertz sera choisie par l'homme de l'art en fonction du traitement à préconiser au patient.

Les sorties du module **(A)** et **(B)** sont directement reliées aux entrées du module **(C)** qui module et amplifie des signaux émis par les modules **(A)** et **(B)**, l'addition des signaux **(figures 7 et 8)** donne un signal pulsé, épuré de toutes composantes continues **(figure 9)** par l'adjonction du condensateur de sortie. La sortie du module **(C)** est directement connectée au conducteur d'émission de l'antenne **(3)**.

Les circuits électroniques **(2)** produisant des signaux électriques impulsionnels à haute fréquence pulsée comprennent ainsi la partie de commande de sélection de fréquences et réglage de puissance (module **(B)**).

Pour des raisons d'optimisation de l'emploi dans les différentes professions de l'esthétique ou de la coiffure, le dispositif peut comprendre aussi un système chauffant par rayonnement ou par air pulsé.

## Revendications

1. Dispositif émetteur d'ondes électromagnétiques à hautes fréquences pulsées à effet athermique, pour le traitement du crâne d'un individu pour des applications médicales, esthétiques ou de soins capillaires, comprenant au moins un circuit **(2)** produisant des signaux électriques impulsionnels à hautes fréquences pulsées, ce circuit étant relié à au moins une antenne **(3)** pour l'émission d'un champ électromagnétique à haute fréquence, l'ensemble étant relié à une source d'alimentation électrique, caractérisé en ce que:
le dispositif comprend un casque de taille à envelopper la boîte crânienne et un boîtier de commande **(5)** comprenant le circuit **(2)** produisant les signaux électriques impulsionnels à hautes fréquences ainsi que la partie de commande de sélection de fréquences et réglage de puissance d'émission;
le casque supporte une ou plusieurs antennes **(3)** pour l'émission du champ électromagnétique à haute fréquence;
le casque est relié à un système mécanique **(4)** ou électrique commandé manuellement ou automatiquement permettant de positionner le casque au niveau de la boîte crânienne sans contact avec celle-ci;
de façon que des ondes électromagnétiques à hautes fréquences pulsées athermiques de fréquence prédéterminée et de faible puissance d'émission peuvent être appliquées et localisées directement sur la zone à traiter, selon le choix de l'homme de l'art.

2. Dispositif suivant la revendication 1, caractérisé en ce que le circuit électronique **(2)** comprend un module d'émission haute fréquence **(A)**, un module basse fréquence **(B)**, un module modulateur et amplificateur **(C)**.

3. Dispositif suivant la revendication 1, caractérisé en ce que la ou les antennes sont réalisées dans un matériau conducteur produisant l'émission du champ électromagnétique de haute fréquence pulsée sans contact avec la zone à traiter.

4. Dispositif suivant la revendication 2 caractérisé en ce que la sortie du module modulateur et amplificateur **(C)** est connectée directement au conducteur d'émission **(3)** de l'antenne.

5. Dispositif suivant la revendication 2 caractérisé en ce que le module **(A)** générateur d'émission haute fréquence est de type sinusoïdal et de fréquence 27.120 Mhz et en ce que les composantes de ce module **(A)** sont directement reliées à l'alimentation, et la sortie de ce module **(A)** est directement reliée à l'une des entrées du module amplificateur et modulateur.

6. Dispositif suivant la revendication 2, caractérisé en ce que le module **(B)** générateur d'émission basse fréquence variable est de type signal carré ayant un rapport cyclique d'environ 50% et en ce que les composantes de ce module **(B)** sont directement reliées à l'alimentation et la sortie de ce module **(B)** directement reliée à l'une des entrées du module amplificateur et modulateur **(C)**.

7. Dispositif suivant la revendication 2, caractérisé en ce que les entrées du module **(C)** amplificateur et modulateur sont reliées directement au module **(A)** générateur d'émission haute fréquence et au module **(B)** générateur basse fréquence additionnant les signaux issus de ceux-ci et pour générer un signal haute fréquence pulsé émis directement sur le conducteur d'émission de l'antenne **(3)**.

8. Dispositif suivant la revendication 2, caractérisé par la présence de plusieurs circuits électroniques **(2)** produisant des signaux électriques impulsionnels à haute fréquence pulsées reliés chacun à leur propre antenne **(3)** pour l'émission d'un champ électromagnétique de haute fréquence.

9. Dispositif suivant la revendication 2, caractérisé en ce que le dispositif comprend aussi un système chauffant par rayonnement ou par air pulsé.

## Claims

1. Device that generates high frequency pulsed electromagnetic waves with athermic effects providing treatment to the scalp of the patient to medical, aesthetical or capillary use. The device includes at least one circuit **(2)** producing pulsed electrical signals of high frequency pulses, connected to at least one antenna **(3)** for a high frequency electromagnetic field transmission, the assembly connected to an electrical source, characterised by the fact that:
the device includes a helmet which can envelop the scalp and a control box **(5)** including the circuit **(2)** producing high frequency pulsed electrical signals as well as the part that commands the selection of frequency and adjustment of the power;
the helmet supports one or more antennae **(3)** for the emission of the high frequency electromagnetic field;
the helmet is connected to a mechanical system **(4)** or an electromechanical system controlled manually or automatically permitting to position the helmet adjacent to the scalp without any contact with it;
so that high frequency pulsed electromagnetic waves with athermic effects with a predetermined frequency and a weak power can be applied and located directly on the affected area according to the consultant choice.

2. Device, according to claim 1, characterised by the fact that the electronic circuit **(2)** includes a high frequency transmission module **(A)**, a low frequency module **(B)**, a module modulator and amplifier **(C)**.

3. Device, according to claim 1, characterised by the fact that the antenna or antennae are made of a conducting material producing the transmission of the pulsed high frequency electromagnetic field without any contact with the affected area.

4. Device, according to claim 2, characterised by the fact that the output of the module modulator and amplifier **(C)** is directly connected to the transmission conductor **(3)** of the antenna.

5. Device, according to claim 2, characterised by the fact that the module **(A)** generator of the high frequency transmission is of a sinusoidal type and a frequency of 27.120 Mhz and the components of this module **(A)** are directly connected to the source, and the output of this module **(A)** is directly connected to one of the inputs of the module amplifier and modulator.

6. Device, according to claim 2, characterised by the fact that the module **(B)** generator of low frequency variable transmission is of a square signal type having a cyclic proportion of approximately 50% and the components of this module **(B)** are directly connected to the source and the output of this module **(B)** is directly connected to one of the inputs of the module amplifier and modulator **(C)**.

7. Device, according to claim 2, characterised by the fact that the inputs of the module **(C)** amplifier and modulator are directly connected to the module **(A)** generator of high frequency transmission and to the module **(B)** generator of low frequency adding the signals coming from them to generate a high frequency pulsed signal transmitted directly to the transmission conductor of the antenna **(3)**.

8. Device, according to claim 2, characterised by the presence of many electronic circuits **(2)** producing pulsed electrical signals of high frequency pulses - each one connected to its own antenna **(3)** to transmit a high frequency electromagnetic field.

9. Device, according to claim 2, characterised by the fact that the device also includes a heating system by radiation or pulsed air.

## Patentansprüche

1. Sendevorrichtung von pulsierenden elektromagnetischen Hochfrequenzwellen mit athermischen Effekten, für die Behandlung der Kopfhaut zur medizinischen, esthetischen Nutzanwendung oder zur kapilaren Pflege, versehen mit mindestens einem Schaltkreis der elektrische pulsierende Hochfrequenzsignalimpulse produziert Dieser ist mit mindestens einer Antenne zur Sendung eines elektromagnetischen Hochfrequenzfeldes verbunden. Das Ganze wird von einer elektrischen Spannung versorgt.
Die Sendevorrichtung enspricht folgenden Charakteristiken:
Zu der Vorrichtung gehört ein Helm, der die gesammte Sehädeldecke umspannt und ein Bedienungspult (5) mit dem dazugehörenden Schaltkreis zur Herstellung elektrischen, pulsierenden Hochfraqenzsignalimpulsen und dem Bedienungselement für Frequenzwahl und die Einstellung der Sendeleistung.
Der Helm ist Träger einer oder mehrerer Antennen (3) zur Sendung des elektromagnetischen Hochfrequenzfeldes.
Der Helm ist an einem mechanischen (4) oder elektrischen System angebracht mit manueller oder automatischer Bedienung, um sie über der Schädeldecke zu positionieren ohne diese zu berühren;
so, daß die pulsierenden, elektromagnetischen und athermischen Hochfrequenzwellen mit vorgewählter Frequenz und geringer Sendeleistung frei nach Wunsch des Beidienungspersonals lokal auf den zu behandelden Bereich angewendet werden können.

2. Die Vorrichtung, der ersten Anforderung entsprechend, ist charakterisiert durch die Tatsache, daß zu dem Hochfrequenzsendemodul (A) ein Niederfrequenzmodul (B) und ein Verstärkermodul (C) gehört.

3. Die Vorrichtung, der ersten Anforderung entsprechend, ist charakterisiert durch die Tatsache, daß die Antenne oder Antennen mit einem leitfähigem Material verbunden sind zur Sendung des elektromagnetischen, pulsierenden Hochfrequenzfeldes ohne den zu behandelnden Bereich zu berühren.

4. Die Vorrichtung, derzweiten Anforderung entsprechend, ist charakterisiert durch die Tatsache, daß der Ausgang des modulierenden und verstärkenden (C) Moduls direkt mit dem Leiter der Sendeantenne (3) verbunden ist

5. Die Vorrichtung, derzweiten Anforderung entsprechend, ist charakterisiert durch die Tatsache, daß das Modul (A) ein Generator zur Hochfrequenzsendung von Sinuswellen ist mit der Frequenz von 27,120 MHz, und daß die Komponenten des Moduls (A) direkt an die Spannungsquelle angeschlossen sind und der Ausgang des Moduls (A) direkt mit einem der Eingänge des verstärkenden und modulierenden Moduls verbunden ist.

6. Die Vorrichtung, der zweiten Anforderung entsprechend, ist erstens charakterisiert durch die Tatsache, daß das Modul (B) ein variabler Niederfrequenzsendegenerator für Rechteckwellen ist, mit einem zyklischen Verhältnis von 50% und zweitens durch die Tatsache, daß die Komponenten des Moduls (B) direkt an die Spannungsquelle und der Ausgang des Moduls (B) dreht an einen der Eingänge des verstärkenden und modulierenden Moduls (C) angeschlossen sind.

7. Die Vorrichtung, der zweiten Anforderung entsprechend, ist erstens charakterisiert durch die Tatsache, daß die Eingänge des verstärktenden und modulierenden Moduls (C) direkt an das Modul (A) angeschlossen sind (Hochfrequenzgenerator) und an das Modul (B) (Niederfrequenzgenerator), der die Signale addiert und ein pulsierendes Hochfrequenzsignal erzeugt und es direkt auf den Leiter der Sendeantenne (3) weitergibt.

8. Die Vorrichtung, der zweiten Anforderung entsprechend, ist charakterisiert durch die Presenz mehrerer elektronischer Schaltkreise (2), die elektrische, pulsierende Hochfrequenzsignalimpulse erzeugen, die alle mit ihrer zur Sendung mitelektromagnetischen Hochfequenzfeldes entsprechenden Antenne (3) verbunden sind.

9. Die Vorrichtung, der zweiten Anforderung entsprechend, kann mit einem Heizsystem (Strahlung oder Luft) ausgestattet sein.
